Europäisches Patentamt

European Patent Office

Office européen des brevets

(11) Publication number: **0 073 755**
**B1**

(12) **EUROPEAN PATENT SPECIFICATION**

(45) Date of publication of patent specification: **18.06.86**

(51) Int. Cl.⁴: **A 61 F 2/16**

(21) Application number: **81900914.3**

(22) Date of filing: **20.03.81**

(86) International application number:
**PCT/US81/00355**

(87) International publication number:
**WO 82/03004 16.09.82 Gazette 82/22**

(54) **FLEXIBLE INTRAOCULAR LENS.**

(30) Priority: **06.03.81 US 241205**

(43) Date of publication of application:
**16.03.83 Bulletin 83/11**

(45) Publication of the grant of the patent:
**18.06.86 Bulletin 86/25**

(84) Designated Contracting States:
**FR**

(56) References cited:
EP-A-0 032 835
FR-A-1 103 399
GB-A-2 057 270
US-A-3 925 825
US-A-4 056 855
US-A-4 073 015
US-A-4 092 743
US-A-4 159 546
US-A-4 174 543
US-A-4 254 511
US-A-4 257 130

British Journal of Ophthalmology, vol. 45, no.
1, Issued January 1961, pages 37-43,
"Experiences with twelve cases of intraocular
anterior chamber implants for aphakia", by J.
Boberg-Ans.

(73) Proprietor: **CooperVision Inc.**
**75 Willow Road**
**Menlo Park California 94304 (US)**

(72) Inventor: **Bayers, Jon H.**
**2935 Bechelli Lane, Suite C**
**Redding, CA 96001 (US)**

(74) Representative: **Finck, Dieter et al**
**Patentanwälte v. Füner, Ebbinghaus, Finck**
**Mariahilfplatz 2 & 3**
**D-8000 München 90 (DE)**

Courier Press, Leamington Spa, England.

## Description

The invention relates to an intraocular lens comprising a lens portion and at least one flexible loop which is fastened to the lens portion and extends along a side of the iris up to an angle of the eye. Such an intraocular lens is known from NL—A—8004782, which corresponds to GB—A—2057270 (not pre-published).

Intraocular lens implantation has evolved into the preferred method of remedying the correction of vision, particularly after cataract removal. Previous lenses supported in the anterior chamber of the eye by contact of loops with the anterior chamber angle are easy to insert, can be used with intracapsular surgery, have an always visible fixation and cannot dislocate posteriorly into the vitreus cavity. However, when the supporting loops are elastically deformed by the forces exerted from the eye to them, the loops tend to bend themselves in a direction normal to the plane of the iris. As a result, the corneal endothelium is touched by the loops or the lens portion, damaging the endothelium seriously.

To avoid endothelial contact, intraocular lenses fixed in the posterior chamber of the eye have been favoured, such as the lens described in the NL—A—8004782. From the lens portion of this intraocular lens support loops extend to the periphery of the posterior chamber, that is more precise the angle of the anterior and posterior capsule walls. However, posterior chamber angle fixation lenses require extracapsular cataract surgery before insertion and are difficult to insert since the surgeon is not able to see the loops during insertion and afterwards. Complications due to improper location of the loops arise frequently.

US—A—4254511 describes an intraocular lens of another kind which is not supported by flexible loops extending into an angle of the eye, but by relatively rigid loops engaging the inner edge of the iris. However, in these iris-engaging clip supported lenses the loops tend to cut into the edge of the iris. Additionally, these lenses are susceptible to dislocation, and chemically induced full dilation of the iris as often requested in post-operative treatment is not possible. To prevent cutting of the iris, the loops have a non-circular, such as oval, cross-sectional profile, the flattened surface of the profile being provided to contact the inner side of the iris. As the loops are relatively small and rigid, the oval profile does not contribute to any flexibility of the loops.

It is the object of the present invention to provide an intraocular lens with fixation visible at any time, yet without endangering the endothelium and without being susceptible to dislocation.

According to the present invention, this object is solved in that in the intraocular lens of the generic kind the material of the loop has a cross-sectional configuration with relative long dimension and vertically thereto a relative short dimension, said long dimension being oriented substantially normal to the plane of the iris.

Preferably, the material of the loop has an oval cross-sectional configuration.

The orientation of the oval cross-section is, according to the invention, opposite to the orientation as given in the US—A—4254511.

The intraocular lens of the present invention employs an optical lens portion which may be placed in the posterior chamber of the eye immediately following cataract removal. The lens portion is designed to be placed adjacent the iris and includes a pair of loops fastened to the lens portion. Each loop includes a first portion which passes through the pupil of the eye and a second portion connected to the first portion which extends along the other side of the iris for contact with the peripheral area of the anterior chamber, namely the angle found between the cornea and iris.

Endothelial touch of the intraocular lens is prevented by the oval cross-sectional configuration of the loops. Any force directed at the end portions of the loops would cause the loops to spread laterally, generally normal to the optical axis of the lens and pupil rather than vertically inwardly or outwardly in relation to the plane of the iris.

In another embodiment the intraocular lens of the present invention may include a lens portion having at least one loop fastened thereto. Such a lens may be placed in the anterior chamber immediately following cataract surgery or at a later surgery. The embodiment could be also placed in the posterior chamber if an extra capsular surgery were performed. The flexibility of the at least one loop would allow for variation in ocular size.

The intraocular lens of the present invention has a lens portion which may be positioned in the posterior chamber of the eye without the necessity of extra capsular cataract surgery, deriving fixation from the anterior chamber of the eye which permits the observation of the actual fixation by the surgeon. The intraocular lens permits the dilation of the iris postoperatively without endangering the fixation of the intraocular lens and provides for proper fixation with all normally sized eyes. The lens portion of the intraocular lens according to the present invention may be positioned in the anterior chamber or in the posterior chamber of the eye. In each embodiment endothelial touch is prevented through the physical structure and configuration of the loops.

Preferred embodiments of the invention are, by way of example, described in more detail with reference to the drawings, in which:

Fig. 1 is a top plan view of an intraocular lens implanted in a human eye,

Fig. 2 is a sectional view showing a first embodiment of the intraocular lens implanted in a human eye,

Fig. 3 is a top plan view of the intraocular lens showing deformation of its loops upon the application of force,

Fig. 4 is a view taken along line 4—4 of Fig. 3,

Fig. 5 is a sectional view of a second embodiment of the intraocular lens placed in the anterior chamber of the eye,

Fig. 6 is a top plan view of the second embodiment of the intraocular lens.

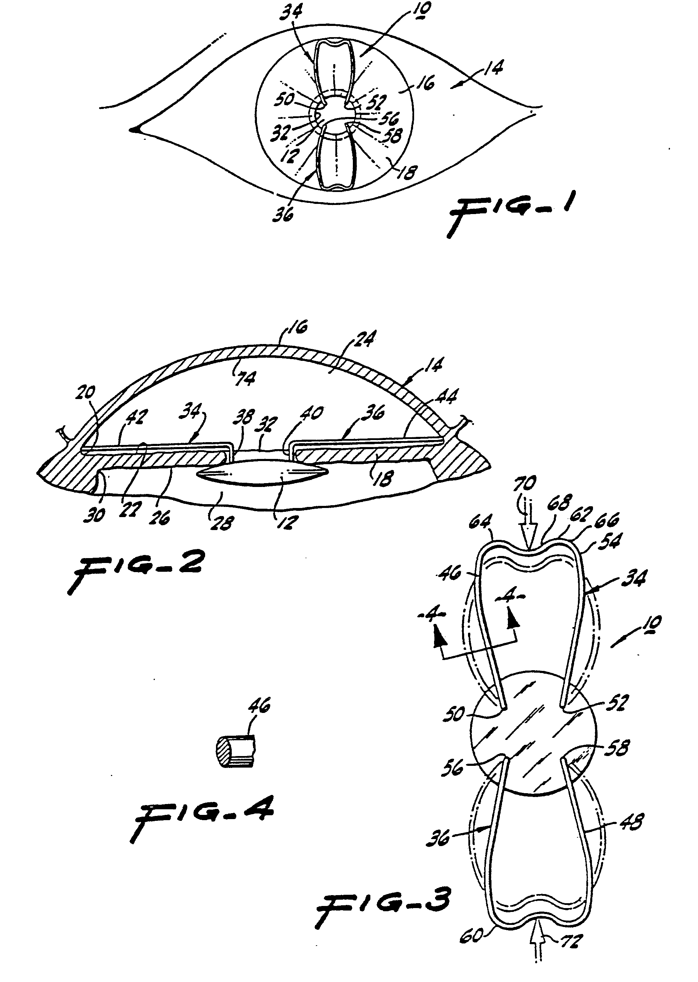

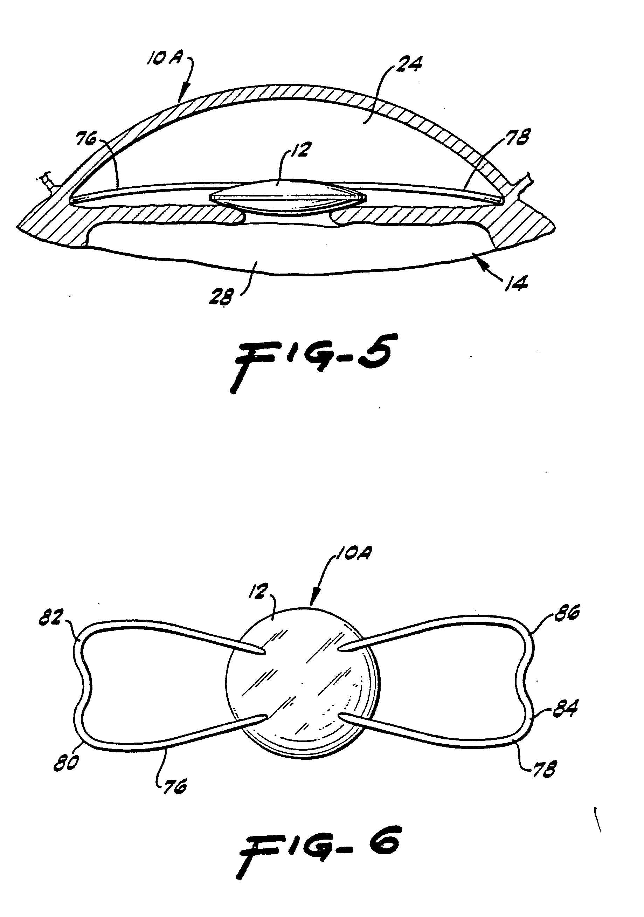

With reference to the drawings, Fig. 1 shows an intraocular lens which is identified herein by reference character 10. The intraocular lens 10 includes a lens portion 12 having an optical axis generally coaxial with the pupillary axis which may be constructed of any suitable material which is nonreactive to human tissue. For example, ophthalmic glass, and plastic such as polymerized methylmethacrylate and the like are adequate. Lens portion 12 may take the form of a convex, plano-convex, or other lens shape necessary to correct the vision of eye 14 immediately following cataract removal. Fig. 2 most clearly shows eye 14 having a cornea 16 and an iris 18 which form an angle 20 therebetween. Iris 18 has a first side 22 lying in anterior chamber 24 and a second side 26 lying in posterior chamber 28. The intraocular lens 10 is shown in Fig. 2 in place within eye 14 following intracapsular cataract surgery. It should be noted that the intraocular lens 10 may be employed to correct aphakia following extra capsular cataract surgery as well. Eye 14 also includes ciliary sulcus 30 where lenses of the prior art are fixed. As can be seen, ciliary sulcus is not visible from the surgeon's point of view outside of cornea 16. Fig. 2 also illustrates the position of pupil 32 formed by iris 18.

The intraocular lens 10 also includes a first loop 34 and a second loop 36 extending from lens portion 12. Although loops 34 and 36 are depicted as identical in the drawings, certain variations as to shape may cause one loop to be formed differently from the other. Each loop 34, 36 includes a first portion 38 and 40 which is fastened to lens portion 12 by gluing, heat or sonic welding, moulding, or any other method known in the art. First portions 38 and 40 pass through pupil 32 of eye 14 and connect to second portions 42 and 44 of loops 34 and 36 respectively. Second portions 42 and 44 extend along side 22 of iris 18 and contact angle 20 of eye 14 at the periphery of eye 14. In contrast, lens portion 12 remains in posterior chamber 28 against side 26 of iris 18. Turning to Fig. 1, it may be seen that loops 34 and 36 may take the form of elongated members 46 and 48, forming a closed loop. In such a case first portion 38 would include a pair of posts 50 and 52, Fig. 1, fastened to lens portion 12, extending through pupil 32, and fastened to continuous member 54 to complete the loop. It should be noted that elongated member 48 also includes a pair of posts 56 and 58 as well as a continuous member 60. It is fully contemplated that first and second loops 34 and 36 may be open rather than closed.

Turning to Fig. 3, it may be seen that at least one loop member 46 may include an end portion 62 having areas 64 and 66 which bulge outwardly in relation to indent portion 68. Thus, areas 64 and 66 of end portion 62 would make contact with angle 20 while indent portion 68 generally would not. Loop 36 may be formed without the bulged areas 64 and 66 of appendage 34 to create a three point fixation. Likewise, as shown in Fig. 3, loop 36 may be formed identically to loop 34 creating a four point fixation at angle 20. Arrows 70 and 72 represent an effective force on loops 34 and 36; the actual force would normally be on the bulged areas of loops 34 and 36, e.g. areas 64 and 66 of loop 34.

Turning to Fig. 4, it may be seen that elongated members 46 and 48 have an oval cross-sectional configuration. As a result of this feature, force 70 and 72 cause loops 34 and 36 to expand laterally across the top of side 22 of iris 18 in a direction generally normal to the optical axis of the lens and pupil rather than upwardly toward cornea 16 and endothelium layer 74. It may be apparent that endothelium layer 74 will not be touched by loops 34 and 36 and therefore will not be damaged by the same postoperatively. Fig. 3 depicts the lateral movement of loops 34 and 36 in phantom.

Fig. 5 shows another intraocular lens embodiment 10A where lens portion 12 includes loops 76 and 78 which extend to the angle 20. It should be noted that lens portion 12 and loops 76 and 78 are located in the anterior chamber 24 of eye 14. It is intended that the intraocular lens 10A may be similarly placed in the posterior chamber 28 between portions of the ciliary sulcus 30. Loops 76 and 78 may be flattened as shown in Fig. 4 to restrict flexure of the same laterally in relation to iris 18 as shown in Fig. 3. Bulge areas 80, 82, 84 and 86, likewise, add purchase to the intraocular lens 10A, Fig. 6.

In operation, the surgeon opens cornea 16 in the usual manner and inserts intraocular lens 10 within eye 14. Loop 34 and loop 36 are placed within the confines of angle 20 and lens portion 12 is forced through pupil 32 which may be dilated to greatly facilitate this maneuver. Intraocular lens 10A is simply placed either in anterior chamber 24 or posterior chamber 28 if the posterior capsule is present, between opposing sides of angle 20 or ciliary sulcus 30 respectively. After insertion, intraocular lens 10 or 10A will not effect any dilation of pupil 32 and the fixation of loops 34 and 36 will always be visible to surgeon.

## Claims

1. An intraocular lens comprising a lens portion (12) and at least one flexible loop (34; 36; 76; 78) which is fastened to the lens portion (12) and extends along a side of the iris (18) up to an angle (20) of the eye, characterized in that the material of the loop (34; 36; 76; 78) has a cross-sectional configuration with relative long dimension and vertically thereto a relative short dimension, said long dimension being oriented substantially normal to the plane of the iris (18).

2. An intraocular lens according to claim 1, characterized in that the material of the loop (34; 36; 76; 78) has an oval cross-sectional configuration.

## Patentansprüche

1. Intraokulare Linse mit einem Linsenteil (12) und wenigstens einer flexiblen Schlaufe (34; 36; 76; 78), die an dem Linsenteil (12) befestigt ist und sich längs einer Seite der Iris (18) bis in einen Augenwinkel (20) erstreckt, dadurch gekennzeichnet, daß das Material der Schlaufe (34; 36; 76; 78) eine Querschnittsform mit einer relativ langen Abmessung und vertikal dazu einer relativ kurzen Abmessung aufweist, wobei die lange Abmessung im wesentlich senkrecht zur Ebene der Iris (18) orientiert ist.

2. Intraokulare Linse nach Anspruch 1, dadurch gekennzeichnet, daß das Material der Schlaufe (34; 36; 76; 78) eine ovale Querschnittsform hat.

## Revendications

1. Une lentille intra-oculaire comprenant une partie-lentille (12) et au moins une boucle flexible (34; 36; 76; 78) qui est fixée sur la partie-lentille (12) et qui s'étend le long d'un côté de l'iris (18) jusqu'à un angle (20) de l'oeil, caractérisée en ce que la matière de la boucle (34; 36; 76; 78) présente une configuration de section droite ayant une dimension relativement longue et, verticalement à celle-ci, une dimension relativement courte, ladite dimension longue étant orientée sensiblement perpendiculaire au plan de l'iris (18).

2. Une lentille intra-oculaire selon la revendication 1, caractérisée en ce que la matière de la boucle (34; 36; 76; 78) présente une section droite de configuration ovale.

FIG-1

FIG-2

FIG-4

FIG-3

FIG-5

FIG-6